# EUROPÄISCHE PATENTANMELDUNG

(11) **EP 2 388 260 A1**
(43) Veröffentlichungstag der Anmeldung: **23.11.2011**
(21) Anmeldenummer: 11003853.6
(22) Anmeldetag: 11.05.2011
(51) Int. Cl.: C07D 413/14

(54) **Herstellungsverfahren für einen Inhibitor eines Blutgerinnungsfaktors**

(30) Priorität: 21.05.2010 DE 102010021188
(71) Anmelder: Archimica GmbH, 65929 Frankfurt am Main (DE)
(72) Erfinder: Nonnenmacher, Michael Dr., 69181 Leimen (DE); Jung, Jörg Dr., 65439 Flörsheim (DE)
(74) Vertreter: Schweitzer, Klaus

(57) **Zusammenfassung**

Die Erfindung betrifft ein Verfahren zur Herstellung von 5-Chlor-N-({(5S)-2-oxo-3-[4-(3-oxo-4-morpholinyl)-phenyl]-1,3-oxazolidin-5-yl}-methyl)-2-thiophencarboxamid (**I**) aus 5-Chlorthiophen-2-carbonsäure und 4-[4-((S)-5-Aminomethyl-2-oxo-oxazolidin-3-yl)-phenyl]-morpholin-3-on (**II**) 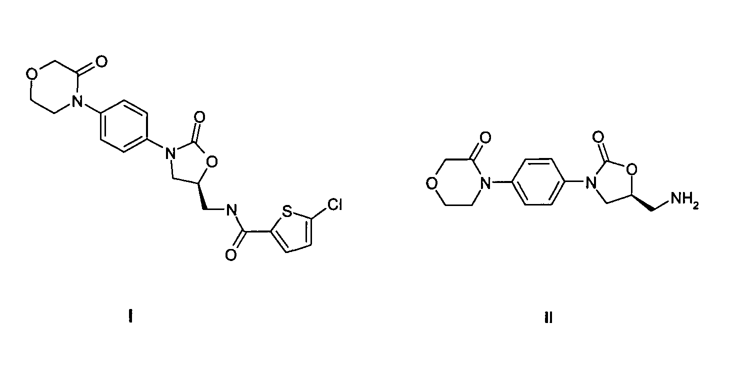 oder einem Salz dieser Verbindung mit einer Mineralsäure oder einer organischen Säure in Gegenwart von Propanphosphonsäure-anhydrid (T3P^{®}).

## Beschreibung

Die vorliegende Erfindung betrifft ein Verfahren zur Herstellung von 5-Chlor-N-({(5S)-2-oxo-3-[4-(3-oxo-4-morpholinyl)-phenyl]-1,3-oxazolidin-5-yl}-methyl)-2-thiophencarboxamid (**I**) aus 5-Chlorthiophen-2-carbonsäure und 4-[4-((S)-4-Aminomethyl-2-oxo-imidazolidin-1-yl)-phenyl]-morpholin-3-on (**II**) bzw. Salzen dieser Verbindung. Die Zielverbindung kann als Inhibitor des Blutgerinnungsfaktors Xa eingesetzt werden.

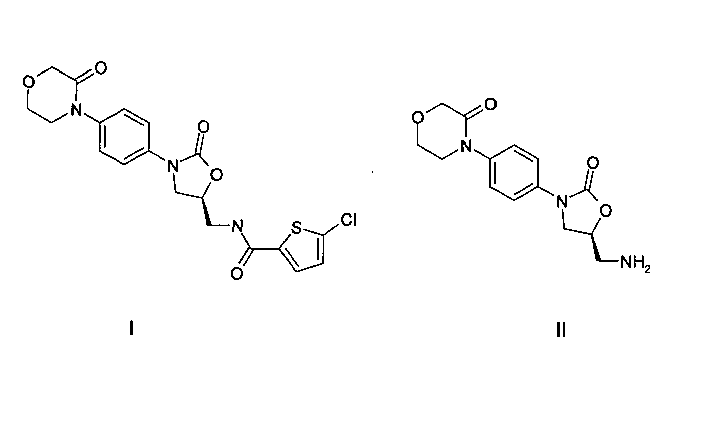

### Stand der Technik

Die Verbindung 5-Chlor-N-({(5S)-2-oxo-3-[4-(3-oxo-4-morpholinyl)-phenyl]-1,3-oxazolidin-5-yl}-methyl)-2-thiophencarboxamid (**I**) wurde in WO01/47919 erstmals beschrieben. Für die Synthese wurden dabei zwei alternative Routen offengelegt. Die erste Alternative geht dabei von einem chiralen Epoxid aus, das nukleophil durch Reaktion mit 4-(4-Aminophenyl)-morpholin-3-on geöffnet und anschließend mit einem Phosgenäquivalent zyklisiert wird (Route B).

Schema 1: Route B zur Herstellung von Verbindung I gemäß Stand der Technik

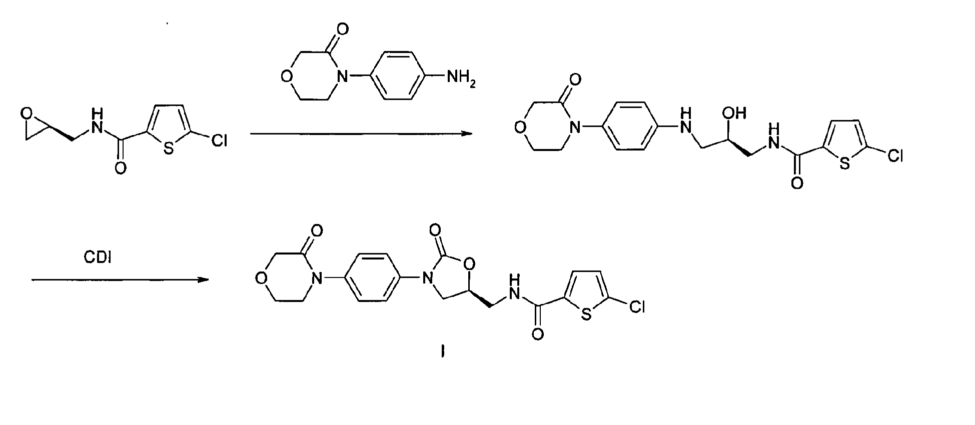

Für die zweite Alternative wird die Zwischenstufe 4-[4-((S)-5-Aminomethyl-2-oxo-oxazolidin-3-yl)-phenyl]-morpholin-3-on (**II**) durch Umsetzung mit 5-Chlorthiophen-2-carbonsäurechlorid oder dem Produkt der Reaktion der freien Carbonsäure mit einer Kombination aus EDCI und HOBT (1-Hydroxy1-H-benzotriazol, N'-(3-Dimethylaminopropyl)-N-ethylcarbodiimid) in das gewünschte Produkt überführt (Route A; der Substituent "X" im Formelschema entspricht einer Abgangsgruppe wie Chlorid oder einer mit dem erwähnten typischen Aktivierungsreagenzien gebildeten). Dabei erfolgt die Umsetzung gemäß WO01/47919 in einem inerten Lösungsmittel in Gegenwart einer Base, in den Beispielen wird z. B. Diisopropylethylamin verwendet.

Schema 1: Alternative Route A zur Herstellung von Verbindung I gemäß Stand der Technik aus Vorstufe II

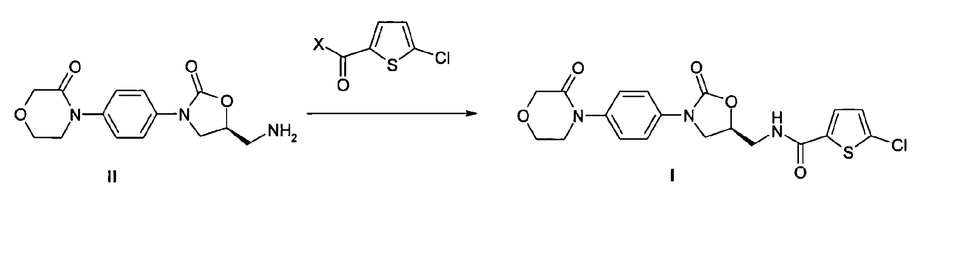

In WO05/068456 wird eine Verbesserung dieser Synthese beschrieben, in der die Umsetzung von 5-Chlorthiophen-2-carbonsäurechlorid mit dem Hydrochlorid der Zwischenstufe II in Gegenwart einer anorganischen Base in einem Ether, Alkohol, Keton, in Wasser oder aber in einem beliebigen Gemisch dieser Lösemittel durchgeführt wird. Darüber hinaus sind weitere Methoden zur Darstellung von 5-Chlor-N-({(5S)-2-oxo-3-[4-(3-oxo-4-morpholinyl)-phenyl]-1,3-oxazolidin-5-yl}-methyl)-2-thiophencarboxamid (**I**) beschrieben worden, die aber nicht das Intermediat 4-[4-((S)-5-Aminomethyl-2-oxo-oxazolidin-3-yl)-phenyl]-morpholin-3-on (**II**) durchlaufen und daher nicht zum Stand der Technik gehören, von dem sich die vorliegende Erfindung abhebt.

Das in WO01/47919 beschriebene Verfahren hat den Nachteil, dass toxische Reagenzien eingesetzt werden und eine technische Umsetzung schwierig ist, während das verbesserte Verfahren aus WO05/068456 auf 5-Chlorthiophen-2-carbonsäurechlorid basiert, was einen zusätzlichen Reaktionsschritt, nämlich die Herstellung des Säurechlorides aus der Carbonsäure bedeutet. Dadurch wird das Verfahren gegenüber einem direkten Zugang aus 5-Chlorthiophen-2-carbonsäure unattraktiv. Für die Chlorierung der Carbonsäure wird zudem stark ätzendes und korrosiv wirkendes Thionylchlorid benötigt. Um die für eine Verwendung als Arzneimittel nötige Reinheit zu erzielen, muß das Produkt der Reaktion außerdem aus Essigsäure umkristallisiert werden.

### Aufgabenstellung

Daraus ergab sich die Aufgabe, ein Verfahren zur Darstellung von 5-Chlor-N-({(5S)-2-oxo-3-[4-(3-oxo-4-morpholinyl)-phenyl]-1,3-oxazolidin-5-yl}-methyl)-2-thiophencarboxamid (**I**) aus 4-[4-((S)-5-Aminomethyl-2-oxo-oxazolidin-3-yl)-phenyl]-morpholin-3-on (**II**) und oder einem geeigneten Salz der Verbindung mit einer Mineralsäure oder organischen Säure der Verbindung zur Verfügung zu stellen, bei dem 5-Chlorthiophen-2-carbonsäure direkt eingesetzt werden kann, das in technischem Maßstab durchführbar ist, auf toxische Lösungsmittel und Reagenzien verzichtet und das Zielprodukt in hoher Reinheit liefert.

Überraschenderweise wurde nun gefunden, dass die synthetische Knüpfung der Amid-Bindung aus 5-Chlorthiophen-2-carbonsäure und der primären Aminofuntkion im 4-[4-((S)-5-Aminomethyl-2-oxo-oxazolidin-3-yl)-phenyl]-morpholin-3-on (**II**) besonders gut in Gegenwart von Propanphosphonsäureanhydrid (T3P^{®}) durchzuführen ist. Dies gilt ebenso, wenn **II** als Salz eingesetzt wird.

Gegenstand der Erfindung ist demnach ein Verfahren zur Umsetzung von 5-Chlorthiophen-2-carbonsäure mit 4-[4-((S)-5-Aminomethyl-2-oxo-oxazolidin-3-yl)-phenyl]-morpholin-3-on (**II**) und oder einem geeigneten Salz der Verbindung mit einer Mineralsäure oder einer organischen Säure zu 5-Chlor-N-({(5S)-2-oxo-3-[4-(3-oxo-4-morpholinyl)-phenyl]-1,3-oxazolidin-5-yl}-methyl)-2-thiophencarboxamid (**I**), dadurch gekennzeichnet, dass die Umsetzung in Gegenwart von Propanphosphonsäureanhydrid (T3P®, CAS No 68957-94-8) durchgeführt wird). Es wird so in nur einem Schritt ein sehr sauberes Produkt erhalten, das durch eine zusätzlich Umkristallisation aus NMP (N-Methylpyrrolidon) weiter gereinigt werden kann und bei dem alle Nebenkomponenten in der HPLC auf ein Niveau von jeweils kleiner als 0,03 % (a/a) gesenkt werden können.

Bevorzugt ist der Einsatz von 5-Chlorthiophen-2-carbonsäure mit 4-[4-((S)-5-Aminomethyl-2-oxo-oxazolidin-3-yl)-phenyl]-morpholin-3-on (**II**) oder 4-[4-((S)-5-Aminomethyl-2-oxo-oxazolidin-3-yl)-phenyl]-morpholin-3-on als Salz einer anorganischen oder organischen Säure, besonders bevorzugt der Einsatz des Hydrochlorids.

Das Kupplungsreagenz Propanphosphonsäureanhydrid, von dem bevorzugt 0,8 bis 2 Äquivalente eingesetzt werden, besonders bevorzugt 1,2 bis 1,8 Äquivalente, bevorzugt eingesetzt als ca. 50%ige Lösung, ermöglicht die erwünschte Reaktion zur Bildung der Amid-Bindung unter sehr milden Bedingungen, bevorzugt in Gegenwart einer organischen Base, bei der es sich in einer besonders bevorzugten Ausführungsform der Erfindung um Triethylamin oder Ethyldiisopropylamin handelt. Dabei werden bevorzugt 1 bis 8 Äquivalente der Base, besonders bevorzugt 3 bis 6 Äquivalente eingesetzt. Des Weiteren kann die Umsetzung in nicht-toxischem Medium erfolgen, bevorzugt in Dialkylethern oder Carbonsäurealkylestern, besonders bevorzugt in Essigsäureethylester.

Das erfindungsgemäße Verfahren zeichnet sich auch dadurch aus, dass die Umsetzung in einem besonders milden Temperaturfenster durchgeführt werden kann, bevorzugt zwischen - 20 und + 80 °C, besonders bevorzugt zwischen - 10 und + 50°C und ganz besonders bevorzugt zwischen 0 °C und Raumtemperatur (+23°C).

Erfindungsgemäß erfolgt die Aufarbeitung vorzugsweise durch Zugabe von Wasser zum Reaktionsgemisch wobei das Produkt als Feststoff anfällt und so in einfacher Weise isoliert werden kann.

Das so erhaltene Produkt kann zum Beispiel durch Lösen in heißem organischem Lösungsmittel, vorzugsweise N-Methylpyrrolidon oder Essigsäure und anschließende Kristallisation durch Abkühlen der Lösung auf vorzugsweise eine Temperatur zwischen 0 °C und +35 °C und vorzugsweise durch Zugabe von reinem 5-Chlor-N-({(5S)-2-oxo-3-[4-(3-oxo-4-morpholinyl)-phenyl]-1,3-oxazolidin-5-yl}-methyl)-2-thiophencarboxamid (**I**) als reines Produkt isoliert werden. Besonders bevorzugt ist die Kristallisation aus NMP, die ein besonders reines Produkt liefert.

Im Folgenden wird die Erfindung durch ein bevorzugtes Ausführungsbeispiel näher beschrieben, nicht jedoch eingeschränkt:

### Beispiele:

### Beispiel 1

Umsetzung von 5-Chlorthiophen-2-carbonsäure mit 4-[4-((S)-4-Aminomethyl-2-oxo-imidazolidin-1-yl)-phenyl]-morpholin-3-on (**II**) zu 5-Chlor-N-({(5S)-2-oxo-3-[4-(3-oxo-4-morpholinyl)-phenyl]-1,3-oxazolidin-5-yl}-methyl)-2-thiophencarboxamid (**I**)

47.0 g 4-[4-((S)-5-Aminomethyl-2-oxo-oxazolidin-3-yl)-phenyl]-morpholin-3-on Hydrochlorid und 28.0 g 5-Chlorthiophen-2-carbonsäure werden zusammen mit 75.0 g Ethyldiisopropylamin in 132 g Ethylacetat vorgelegt und auf 10 °C gekühlt. Nun werden 125 g einer 50 %igen Lösung von Propanphosphonsäureanhydrid (T3P^{®}) in Ethylacetat über einen Zeitraum von ca. 30 Minuten zudosiert. Dabei wird die Temperatur bei 10-15 °C gehalten. Es wird noch für ca. 12 h weiter gerührt und dabei die Temperatur langsam auf 25 °C angehoben. Anschließend gibt man 210 g Wasser tropfenweise über einen Zeitraum von ca. 60 Minuten zu. Die mehrphasige Mischung wird noch 60 Minuten gerührt und anschließend abgesaugt. Das Rohprodukt wird mit 100 g Wasser und anschließend mit 100 g Aceton gewaschen und anschließend getrocknet. Es werden 58.9 g (94 % d. Th.) 5-Chlor-N-({(5S)-2-oxo-3-[4-(3-oxo-4-morpholinyl)-phenyl]-1,3-oxazolidin-5-yl}-methyl)-2-thiophencarboxamid (**I**) erhalten. Die Reinheit des so erhaltenen Produktes ist > 98 % Flächenprozent (HPLC)

### Beispiel 2

Umkristallisation von 5-Chlor-N-({(5S)-2-oxo-3-[4-(3-oxo-4-morpholinyl)-phenyl]-1,3-oxazolidin-5-yl}-methyl)-2-thiophencarboxamid (**I**)

60.0 g des nach Beispiel 1 erhaltenen Produktes werden in 300 g NMP suspendiert und die Mischung wird auf 100 °C erhitzt. Man rührt für 10 bis 20 Minuten und lässt danach langsam über einen Zeitraum von 1,5 Stunden auf Raumtemperatur abkühlen. Danach lässt man noch für einen Zeitraum von 30 Minuten weiterrühren und saugt anschließend das Produkt ab. Der Filterkuchen wird noch mit 300 g Wasser gewaschen und anschließend bis zur Gewichtskonstanz getrocknet. Man erhält 54.3 g Produkt mit einer Reinheit von 99.9 % Flächenprozent (HPLC), die größte einzelne Verunreinigung ist nach der Umarbeitung kleiner 0.05 % (HPLC, a/a).

Dies entspricht 87 % d. Th. (gesamt über Reaktion und Umkristallisation) und 92 % Umkristallisationsausbeute.

## Patentansprüche

1. Verfahren zur Herstellung von 5-Chlor-N-({(5S)-2-oxo-3-[4-(3-oxo-4-morpholinyl)-phenyl]-1,3-oxazolidin-5-yl}-methyl)-2-thiophencarboxamid (**I**) aus 5-Chlorthiophen-2-carbonsäure und 4-[4-((S)-5-Aminomethyl-2-oxo-oxazolidin-3-yl)-phenyl]-morpholin-3-on (**II**) 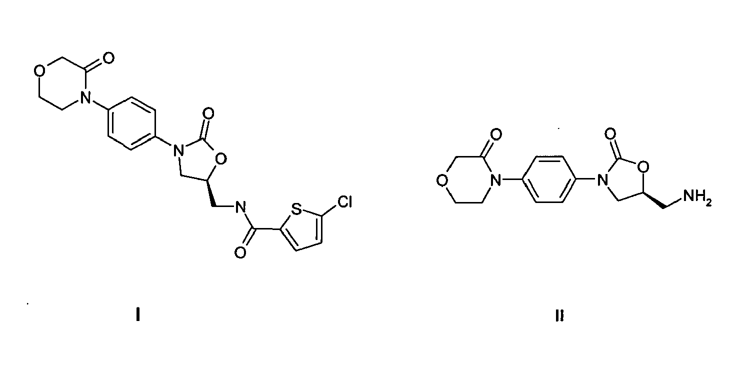 oder einem Salz dieser Verbindung mit einer Mineralsäure oder einer organischen Säure in Gegenwart von Propanphosphonsäure-anhydrid (T3P^{®}).

2. Verfahren nach Anspruch 1, **dadurch gekennzeichnet, dass** die Umsetzung in Gegenwart einer organischen Base durchgeführt wird.

3. Verfahren gemäß einem oder mehreren der Ansprüche 1 oder 2, **dadurch gekennzeichnet, dass** die Umsetzung in Gegenwart eines tertiären Amins durchgeführt wird.

4. Verfahren gemäß einem oder mehreren der Ansprüche 1 oder 3, **dadurch gekennzeichnet, dass** die Umsetzung in Gegenwart von Triethylamin, N-Methylmorpholin oder Ethyldiisopropylamin durchgeführt wird.

5. Ein Verfahren gemäß einem oder mehreren der Ansprüche 1 bis 4 **dadurch gekennzeichnet, dass** 5-Chlorthiophen-2-carbonsäure mit 4-[4-((S)-5-Aminomethyl-2-oxo-oxazolidin-3-yl)-phenyl]-morpholin-3-on (**II**) umgesetzt wird.

6. Ein Verfahren gemäß einem oder mehreren der Ansprüche 1 bis 4 **dadurch gekennzeichnet, dass** 5-Chlorthiophen-2-carbonsäure mit 4-[4-((S)-5-Aminomethyl-2-oxo-oxazolidin-3-yl)-phenyl]-morpholin-3-on Hydrochlorid umgesetzt wird.

7. Ein Verfahren gemäß einem oder mehreren der Ansprüche 1 bis 6 **dadurch gekennzeichnet, dass** die Umsetzung bei einer Temperatur zwischen -20 und +50 °C durchgeführt wird.

8. Ein Verfahren gemäß einem oder mehreren der Ansprüche 1 bis 7, **dadurch gekennzeichnet, dass** die Umsetzung in einem organischen Lösungsmittel aus der Gruppe der Dialkylether oder Carbonsäurealkylester durchgeführt wird.

9. Ein Verfahren gemäß einem oder mehreren der Ansprüche 1 bis 8 **dadurch gekennzeichnet, dass** die Umsetzung in Ethylacetat durchgeführt wird

10. Ein Verfahren gemäß einem oder mehreren der Ansprüche 1 bis 9, **dadurch gekennzeichnet, dass** das Propanphosphonsäureanhydrid als Lösung in Ethylacetat eingesetzt wird.

11. Ein Verfahren gemäß einem oder mehreren der Ansprüche 1 bis 10, **dadurch gekennzeichnet, dass** das aus der Reaktion erhaltene Produkt zur Reinigung aus NMP oder einer Mischung aus NMP mit einem beliebigen anderen Lösemittel mit einem NMP-Anteil von mehr als 60 % umkristallisiert wird.
